# EUROPEAN PATENT SPECIFICATION

(11) **EP 3 300 730 B1**
(45) Date of publication and mention of the grant of the patent: **03.04.2024**
(21) Application number: 17001178.7
(22) Date of filing: 11.07.2017
(51) Int. Cl.: A61K 31/352, A61P 25/28

(54) **THE MEDICAL USE OF 5,7-DIHYDROXY-3-(4-HYDROXYPHENYL)-4H-1-BENZOPIRAN-4-ONE IN THE THERAPY OF HUNTINGTON DISEASE AND PHARMACEUTICALLY ACCEPTABLE FORM OF THE DRUG**
MEDIZINISCHE VERWENDUNG VON 5,7-DIHYDROXY-3-(4-HYDROXYPHENYL)-4H-1-BENZOPIRAN-4-ON BEI DER BEHANDLUNG DER HUNTINGTON-KRANKHEIT UND PHARMAZEUTISCH AKZEPTABLE FORM DES WIRKSTOFFS
UTILISATION MÉDICALE DE 5,7-DIHYDROXY-3-(4-HYDROXYPHÉNYL)-4H-1-BENZOPIRAN-4-ONE DANS LA THÉRAPIE DE LA MALADIE DE HUNTINGTON ET FORME PHARMACEUTIQUEMENT ACCEPTABLE DU MÉDICAMENT

(30) Priority: 15.07.2016 PL 41798316
(43) Date of publication of application: 04.04.2018
(73) Proprietor: Uniwersytet Gdanski, 80-309 Gdansk (PL)
(72) Inventor: Wegrzyn, Grzegorz, 80-299 Gdansk (PL); Baranska, Sylwia, 80-288 Gdansk (PL); Hac, Aleksandra, 80-288 Gdansk (PL); Pierzynowska, Karolina, 80-281 Gdansk (PL)
(74) Representative: Czarnik, Maciej

(56) References cited:
- WO-A2-2007/062186
- MENZE ESTHER T ET AL: "Genistein improves sensorimotor gating: Mechanisms related to its neuroprotective effects on the striatum", NEUROPHARMACOLOGY, vol. 105, 5 January 2016 (2016-01-05), pages 35-46, XP029549458, ISSN: 0028-3908, DOI: 10.1016/J.NEUROPHARM.2016.01.007
- ESTHER T. MENZE ET AL: "Genistein Improves 3-NPA-Induced Memory Impairment in Ovariectomized Rats: Impact of Its Antioxidant, Anti-Inflammatory and Acetylcholinesterase Modulatory Properties", PLOS ONE, vol. 10, no. 2, 12 February 2015 (2015-02-12), page e0117223, XP055452786, DOI: 10.1371/journal.pone.0117223
- KATHRIN PALLAUF ET AL: "Autophagy, polyphenols and healthy ageing", AGEING RESEARCH REVIEWS, vol. 12, no. 1, 1 January 2013 (2013-01-01), pages 237-252, XP055453062, NL ISSN: 1568-1637, DOI: 10.1016/j.arr.2012.03.008
- RAVIKUMAR B ET AL: "Inhibition of mTOR induces autophagy and reduces toxicity of polyglutamine expansions in fly and mouse models of Huntington disease", NATURE GENETICS, NATURE PUBLISHING GROUP, NEW YORK, US, vol. 36, no. 6, 16 May 2004 (2004-05-16), pages 585-595, XP002378584, ISSN: 1061-4036, DOI: 10.1038/NG1362
- ANASTASIUS N ET AL: "Evidence that low-dose, long-term genistein treatment inhibits oestradiol-stimulated growth in MCF-7 cells by down-regulation of the PI3-kinase/Akt signalling pathway", JOURNAL OF STEROID BIOCHEMISTRY AND MOLECULAR BIOLOGY, ELSEVIER SCIENCE LTD., OXFORD, GB, vol. 116, no. 1-2, 1 August 2009 (2009-08-01), pages 50-55, XP026211793, ISSN: 0960-0760, DOI: 10.1016/J.JSBMB.2009.04.009 [retrieved on 2009-05-03]
- Robert E Smith ET AL: "Deregulation of PI3K/Akt/mTOR Signaling Pathways by Isoflavones and its Implication in Cancer Treatment", Cancer Agents in Medicinal Chemistry, 4 April 2013 (2013-04-04), pages 1014-1024, XP055453091, SAIF Zone ISBN: 978-1-60805-154-0 Retrieved from the Internet: URL:http://www.eurekaselect.com/CDN/downlo ad.php?param=Sk9VfUk5dBTFgMvQtkVOhL0FbDQUf 1DLizEzpLzcrvMDiAwNB1cugcGRdmfH8xhcpHBsgaW NxhdGllvbei9wnZGZl8fDdUzMgjc23ZGJhkN2sVmNg mNjlMzRikYWuJiYC2I1lOTNflNGAZkMsGNlh&key=V WlxuRWlguZEcUtQCVNEoc2RDZZkcVJQDTMykRDMD1R jyc1N8DV24LTQ33MzeItMujM16NjQ7zMjhNERD2VE4 eATcVY9TcV [retrieved on 2018-02-21]
- QIANG QIANG ET AL: "Genistein, a natural product derived from soybeans, ameliorates polyglutamine-mediated motor neuron disease", JOURNAL OF NEUROCHEMISTRY, vol. 126, no. 1, 27 February 2013 (2013-02-27), pages 122-130, XP055453007, NEW YORK, NY, US ISSN: 0022-3042, DOI: 10.1111/jnc.12172
- WEILIANG LIAO ET AL: "The Effect of Genistein on the Content and Activity of [alpha]- and [beta]-Secretase and Protein Kinase C in A[beta]-Injured Hippocampal Neurons", BASIC & CLINICAL PHARMACOLOGY & TOXICOLOGY, vol. 112, no. 3, 1 November 2012 (2012-11-01), pages 182-185, XP055453000, COPENHAGEN, DK ISSN: 1742-7835, DOI: 10.1111/bcpt.12009
- Marta Moskot ET AL: "The Phytoestrogen Genistein Modulates Lysosomal Metabolism and Transcription Factor EB (TFEB) Activation", Journal of Biological Chemistry, vol. 289, no. 24, 13 June 2014 (2014-06-13), pages 17054-17069, XP055558231, US ISSN: 0021-9258, DOI: 10.1074/jbc.M114.555300
- Marta Moskot ET AL: "Modulation of expression of genes involved in glycosaminoglycan metabolism and lysosome biogenesis by flavonoids", Scientific Reports, vol. 5, no. 1, 23 March 2015 (2015-03-23), XP055558277, DOI: 10.1038/srep09378

## Description

The subject of the invention 5,7-dihydroxy-3-(4-hydroxyphenyl)-4H-1-benzopiran-4-one and pharmaceutical composition with 5,7-dihydroxy-3-(4-hydroxyphenyl)-4H-1-benzopiran-4-one for use in the treatment of Huntington's disease causing causing reduction of the level of mutant huntingtin protein aggregates in cells by induction of autophagy process thus, inhibiting Huntington's disease.

Huntington's disease (HD) belongs to genetic neurodegenerative disorders. Its prevalence is 1 per 15,000 persons. This disease is inherited in the autosomal dominant manner. Its primary cause is mutation in the *HTT* (*IT15*) gene, coding for the huntingtin protein. This mutation consists of expansion of CAG triplets which leads to formation of a long path (over 40) of glutamine residues (so called the poly-Q sequence). Mutated form of the huntingtin protein tends to form protein aggregates in cells [Walker, 2007 Huntington's disease. Lancet, 369: 218-228

]. Accumulation of such aggregates leads to dysfunction of neurons and progressive neurodegeneration of some brain regions, which results in disturbed movement coordination and dementia. The mutated alleles are maintained in the population due to a late onset of the disease symptoms which occur usually at the end of third or in the fourth decade [Gil and Rego, 2008 Mechanisms of neurodegeneration in Huntington's disease. Eur. J. Neurosci., 27: 2803-2820

WO2007062186 concerns the use of topoisomerase inhibitors in potential treatment of Huntington's disease.

From publication of Esther T Menze et. al: Genistein improves sensorimotor gating: Mechanisms related to its neuroprotective effects on the striatum, Neuropharmacology. 2016 Jun;105:35-46; and Esther T. Menze et. al: Genistein Improves 3-NPA-Induced Memory Impairment in Ovariectomized Rats: Impact of Its Antioxidant, Anti-Inflammatory and Acetylcholinesterase Modulatory Properties, PLoS ONE 10(2): e0117223. doi:10.1371/journal; it is known studies on a symptomatic model of Huntington's disease. In this model, 3-nitropropionic acid (3-NPA) causes appearance of symptoms mimicking Huntington disease in animals. while 3-NPA does not induce the primary cause of Huntington's disease, i.e. accumulation of aggregates of mutant huntingtin - mail goal according to the new mechanism of action according to the invention. No such aggregates occur in cells of animals treated by 3-NPA. Therefore, action of genistein described there is most probably related to its antioxidative properties and enable alleviation of symptoms in the animal model which were caused by completely different mechanism - 3-NPA toxicity - and that event not at least reflects action in natural Huntington's disease pathophysiology process. In publication of Kathrin Pallauf et. al: Autophagy, polyphenols and healthy ageing, Ageing Res Rev. 2013 Jan;12(1):237-52, the author only suggest that autophagy is impaired in Huntington disease without basis for this.

From publication of Brinda Ravikumar et al: Inhibition of mTOR induces autophagy and reduces toxicity of polyglutamine expansions in fly and mouse models of Huntington disease, Nat Genet. 2004 Jun;36(6):585-95 describes experiments in which inhibition of mTOR by rapamycin was used in Huntington's disease models.

Publication of Nitharnie Anastasius etl al: Evidence that low-dose, long-term genistein treatment inhibits oestradiol-stimulated growth in MCF-7 cells by down-regulation of the PI3-kinase/Akt signalling pathway, J Steroid Biochem Mol Biol. 2009 Aug;116(1-2):50-5 and Aamir Ahmad et al: Deregulation of PI3K/Akt/mTOR signaling pathways by isoflavones and its implication in cancer treatment Med Chem. 2013 Sep;13(7):1014-24 are focused on PI3K-Act signaling that is very complicated. Experiments described in D6 and D7 were performed with cancer cells.

Publication of Qiang Qiang etl al: Genistein, a natural product derived from soybeans, ameliorates polyglutamine-mediated motor neuron disease, J Neurochem. 2013 Jul;126(1):122-30 concerns spinal and bulbar muscular atrophy (SBMA). SBMA is a completely different disease than Huntington's disease.

Publication of Weiliang Liao et al: The effect of genistein on the content and activity of α- and β-secretase and protein kinase C in Aβ-injured hippocampal neurons Basic Clin Pharmacol Toxicol. 2013 Mar;112(3):182-5 only suggests that genistein may influence levels of insoluble beta-amyloid.

Publication of Marta Moskot et al: The Phytoestrogen Genistein Modulates Lysosomal Metabolism and Transcription Factor EB (TFEB) Activation, JBC Papers in Press, April 25, 2014 and Marta Moskot et al: Modulation of expression of genes involved in glycosaminoglycan metabolism and lysosome biogenesis by flavonoids describe 5,7-dihydroxy-3-(4-hydroxyphenyl)-4H-1-benzopyran-4-one) in modulated gene network regulating lycosaminoglycans biosynthesis and degradation. Regulatory network linking genistein-mediated control of transcription factor EB (TFEB) gene expression, TFEB nuclear translocation, and activation of TFEB-dependent lysosome biogenesis to lysosomal metabolism id discussed.

Until now, no effective treatment of Huntington's disease is available, and the therapy is restricted to alleviation of some symptoms, like over-activity, personality changes and depression.

Unexpectedly it appeared that genistein (5,7-dihydroxy-3-(4-hydroxyphenyl)-4H-1-benzopiran-4-one), the compound from the group of isoflavones (naturally occurring in plants in a free form (aglycane) or more often in a form of a glycoside (genistin)), called also 4',5,7-trihydroxyisoflavone [Dixon and Ferreira, 2002; Nielsen and Williamson, 2007], causes reduction of the level of mutated huntingtin in cultures of human cells.

The invention relates to 5,7-dihydroxy-3-(4-hydroxyphenyl)-4H-1-benzopiran-4-one for use in the treatment of Huntington's disease causing reduction of the level of mutant huntingtin protein aggregates in cells by induction of autophagy process.

The invention relates to pharmaceutical composition contacting the active compound, 5,7-dihydroxy-3-(4-hydroxyphenyl)-4H-1-benzopiran-4-one, coupled with at least one carrier or pharmaceutical solvent for use in treatment of Huntington's disease. In embodiment dose of 5-150 mg/kg/day and oral administration was presented

### Figure legends:

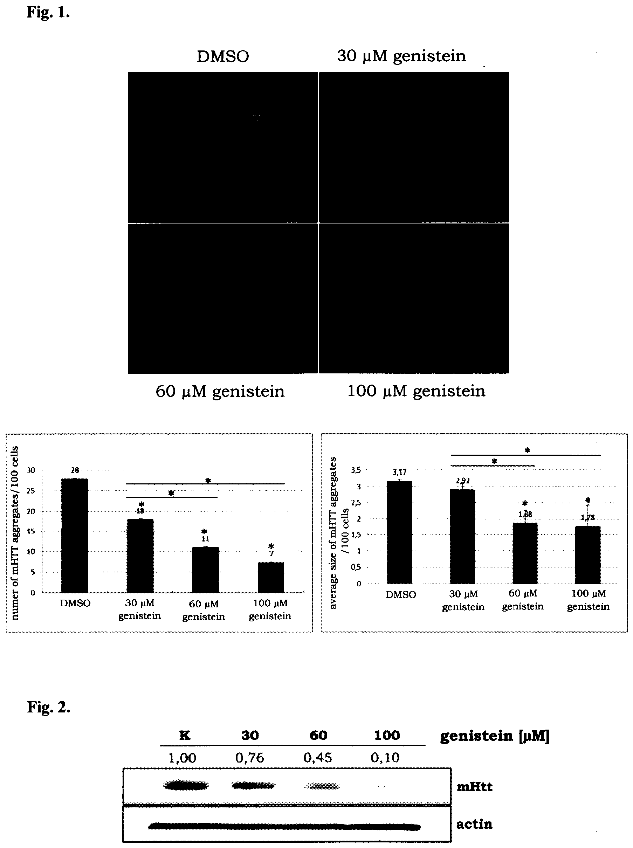

Fig. 1. Number and average size of huntingtin aggregates in human embryonic kidney cells transfected with plasmid pEGFP-Q74 (bearing the 1^{st} exon of the *HTT* gene with 74 CAG repeats), treated with genistein (30, 60 and 100 µM) or DMSO (control cells), in a volume equal to that of genistein, for 48 h. Error bars indicate standard deviation. Statistical analysis was performed with the t-Student test. The differences were considered significant atp<0.05.
Fig. 2. Relative level of mutated huntingtin in human embryonic kidney cells transfected with plasmid pEGFP-Q74 (bearing the 1^{st} exon of the *HTT* gene with 74 CAG repeats), treated with genistein (30, 60 and 100 µM) or DMSO (control cells), in a volume equal to that of genistein, for 96 h. Actin was used as an internal control.

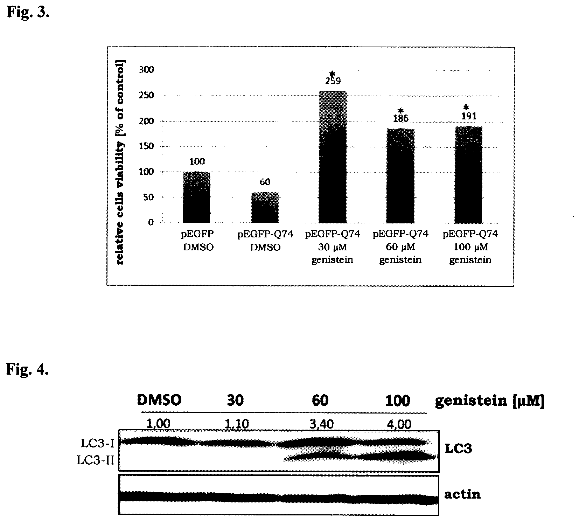

Fig. 3. Relative survival of human embryonic kidney cells transfected with plasmid pEGFP-Q74 (bearing the 1^{st} exon of the *HTT* gene with 74 CAG repeats), treated with genistein (30, 60 and 100 µM) or DMSO (control cells), in a volume equal to that of genistein, for 48 h. In control experiments, a plasmid vector coding for EGFP was used. Statistical analysis was performed with the t-Student test. The differences were considered significant atp<0.05.
Fig. 4. Relative level of the LC3-II protein in human embryonic kidney cells transfected with plasmid pEGFP-Q74 (bearing the 1^{st} exon of the *HTT* gene with 74 CAG repeats), treated with genistein (30, 60 and 100 µM) or DMSO (control cells), in a volume equal to that of genistein, for 96 h. Actin was used as an internal control. Intensity of bands was estimated using the Quantity One software.

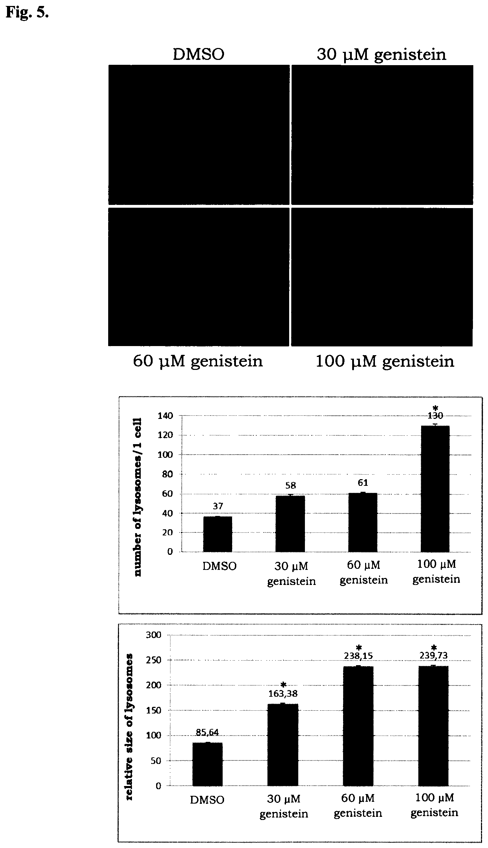

Fig. 5. Number (calculated per one cell) and volume of acidic organelles in human embryonic kidney cells transfected with plasmid pEGFP-Q74 (bearing the 1^{st} exon of the *HTT* gene with 74 CAG repeats), treated with genistein (30, 60 and 100 µM) or DMSO (control cells), in a volume equal to that of genistein, for 96 h. Acidic organelles were labelled by a fluorescent dye LysoTracker Red, while nuclei were marked by DAPI dye. Error bars indicate standard deviation. Statistical analysis was performed with the t-Student test. The differences were considered significant atp<0.05.

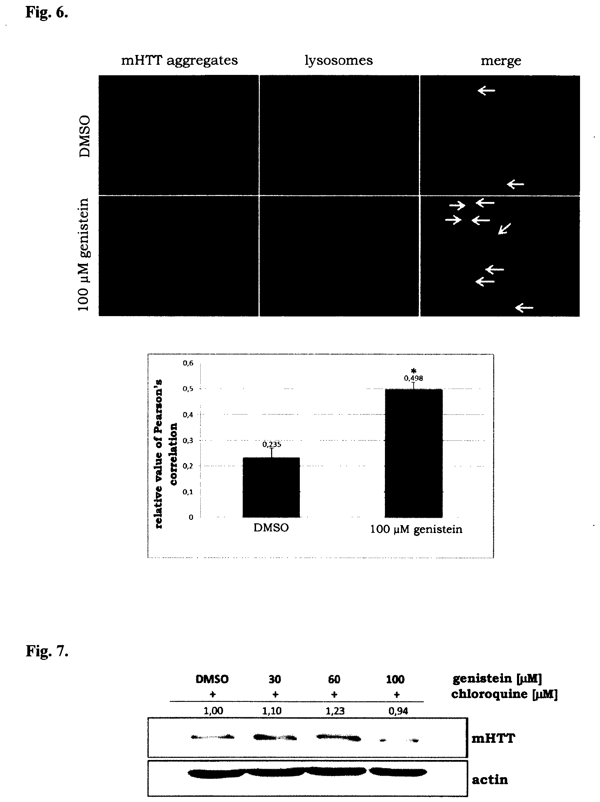

Fig. 6. Co-localization of huntingtin aggregates and lysosomes in human embryonic kidney cells transfected with plasmid pEGFP-Q74 (bearing the 1^{st} exon of the *HTT* gene with 74 CAG repeats), treated with genistein (30, 60 and 100 µM) or DMSO (control cells), in a volume equal to that of genistein, for 96 h. Acidic organelles were labelled by a fluorescent dye LysoTracker Red. The co-localization Pearson factor was calculated by using the ImageJ software. Error bars indicate standard deviation. Statistical analysis was performed with the t-Student test. The differences were considered significant atp<0.05.
Fig. 7. Relative level of mutated huntingtin in human embryonic kidney cells transfected with plasmid pEGFP-Q74 (bearing the 1^{st} exon of the *HTT* gene with 74 CAG repeats), treated with genistein (30, 60 and 100 µM) or DMSO (control cells), in a volume equal to that of genistein, in the presence of 10 µM chloroquine, for 96 h. Actin was used as an internal control.

The invention has been demonstrated in the examples presented below which do not restrict the invention.

### Examples:

In experiments, the model of human embryonic kidney cells (HEK293) transfected with plasmid pEGFP-Q74 bearing the 1^{st} exon of the *HTT* gene with 74 CAG repeats. The product of this gene forms aggregates which cannot be degraded by natural processes, as seen under fluorescent microscope (Fig. 1). Transfected cells were treated with genistein at final concentrations of 30, 60 and 100 µM or with DMSO in a volume equal to that of genistein, for 48 or 96 h. Such cells were observed under fluorescent microscope. Moreover, Western-blotting experiments were performed in order to estimate the level of mutated huntingtin (Fig. 2). In order to assess whether a decrease in the level of mutated huntingtin and reduction of the number and size of huntingtin aggregates can lead to increased cell survival, the viability tests were performed. Cells transfected with plasmid pEGFP-Q74 were treated with genistein and viability was assessed by using the MTT test (Fig. 3).

In order to understand the mechanism of genistein action, experiments were performed to answer the question if this compound can induce the autophagy process. Autophagy is one of the major pathways of degradation of misfolded proteins in cells. Immunodetection of the LC3-II protein, the best investigated marker of autophagy, was performed. Human embryonic kidney cells were transfected with the plasmid, as described above, and were treated with genistein at final concentrations 30, 60 and 100 µM or with DMSO (control cells) in a volume equal to that of genistein, for 96 h. Immunodetection was performed using Wstern-blotting. The results indicated a genistein-concentration-dependent increase in the LC3-II protein amount. Its level, at the highest genistein concentration, was about 4-fold higher than in control experiment (Fig. 4).

Induction of the autophagy process is linked to an increased number of lysosomes. Therefore, number of lysosomes was monitored in experiments with the use of a fluorescent dye, LysoTracker Red. Nuclei were stained with a fluorescent dye DAPI. The microscopic pictures were analyzed with the use of the ImageJ software. The results indicated that increased concentrations of genistein resulted in higher number and volume of acid organelles. This increase is visible at the lowest genistein concentration (30 µM), though the difference relative to the control is significant at 100 µM genistein. An increase in the volume of acid organelles was evident even at 30 µM genistein (Fig. 5).

In order to test whether mutant huntingtin is transported into lysosomes for degradation, co-localization of aggregates of mutated huntingtin with lysosomes was assessed using a fluorescent microscope (Fig. 6). Partial co-localization could be observed even in control experiments (with DMSO). However, its level increased significantly after treatment with 100 µM genistein. Statistical analysis, including Pearson correlation co-efficient, indicated a significant increase in the co-localization in genistein-treated cells. Therefore, mutated huntingtin is delivered into lysosome where it is subsequently degraded.

To test if genistein-induced lysosomal pathway of protein degradation is responsible for removal of mutated huntingtin, experiments were performed in which lysosomal function was inhibited in cells by addition of chloroquine. This compound increases pH inside these organelles, thus, inactivating lysosomal enzymes. Cells were treated with increased concentrations of genistein or with DMSO equal to that of genistein, for 96 h. Immunodetection of mutated huntingtin was performed using Western-blotting (Fig. 7). The results indicated that after inhibition of lysosomal enzymes by 10 µM chloroquine, the level of mutated huntingtin did not change significantly in the presence of genistein. This confirms that activity of lysosomes is responsible for degradation of the mutated form of this protein.

The obtained results indicate that genistein action:
1. leads to decrease in both size and number of mutant huntingtin aggregates in cells (Fig. 1),
2. significantly reduces the level of mutant huntingtin in cells (Fig. 2),
3. causes an increase in viability of cells (Fig. 3),
4. induces the autophagy process (Fig. 4),
5. activates the lysosomal pathway of removing of mutant huntingtin (Figs. 5-7).
The results described above indicate that genistein is a potential drug for Huntington's disease which decreases not only size and number of mutant huntingtin aggregates but also total amount of the mutant protein that is the major cause of neurodegeneration, observed in this disease, thus leading to increased viability of cells.

## Claims

1. 5,7-dihydroxy-3-(4-hydroxyphenyl)-4H-1-benzopiran-4-one for use in the treatment of Huntington's disease causing reduction of the level of mutant huntingtin protein aggregates in cells by induction of autophagy process .

2. 5,7-dihydroxy-3-(4-hydroxyphenyl)-4H-1-benzopiran-4-one for use in the treatment of Huntington's disease according to the claim 1, **characterized in that**, the mechanism of treatment cause an increase in cell viability through considerable reduction of the level of mutant huntingtin in cells.

3. Pharmaceutical composition containing an active compound 5,7-dihydroxy-3-(4-hydroxyphenyl)-4H-1-benzopiran-4-one coupled with at least one carrier or pharmaceutical solvent for use in the treatment of Huntington's disease causing reduction of the level of mutant huntingtin protein aggregates in cells by induction of autophagy process.

4. The composition according to the claim 3 for use in the dose of 5-150 mg/kg/day.

5. The composition according to the claim 4 for use in oral administration.

## Patentansprüche

1. 5,7-Dihydroxy-3-(4-hydroxyphenyl)-4H-1-benzopiran-4-on zur Verwendung bei der Behandlung der Huntington-Krankheit, die eine Verringerung der Menge an mutierten Huntingtin-Proteinaggregaten in Zellen durch Induktion des Autophagie-Prozesses bewirkt.

2. 5,7-Dihydroxy-3-(4-hydroxyphenyl)-4H-1-benzopiran-4-on zur Verwendung bei der Behandlung der Huntington-Krankheit nach Anspruch 1, **dadurch gekennzeichnet, dass** der Behandlungsmechanismus eine Erhöhung der Lebensfähigkeit der Zellen durch eine beträchtliche Verringerung des Niveaus von mutiertem Huntingtin in den Zellen bewirkt.

3. Pharmazeutische Zusammensetzung, enthaltend einen Wirkstoff 5,7-Dihydroxy-3-(4-hydroxyphenyl)-4H-1-benzopiran-4-on, gekoppelt mit mindestens einem Träger oder pharmazeutischen Lösungsmittel, zur Verwendung bei der Behandlung der Huntington-Krankheit, die eine Verringerung der Menge an mutierten Huntingtin-Proteinaggregaten in Zellen durch Induktion des Autophagie-Prozesses bewirkt.

4. Die Zusammensetzung nach Anspruch 3 zur Verwendung in einer Dosis von 5-150 mg/kg/Tag.

5. Die Zusammensetzung gemäß Anspruch 4 zur Verwendung bei oraler Verabreichung.

## Revendications

1. 5,7-dihydroxy-3-(4-hydroxyphényl)-4H-1-benzopiran-4-one destiné à être utilisé dans le traitement de la maladie de Huntington, entraînant une réduction du niveau d'agrégats de protéine huntingtine mutante dans les cellules par l'induction du processus d'autophagie.

2. 5,7-dihydroxy-3-(4-hydroxyphényl)-4H-1-benzopiran-4-one pour utilisation dans le traitement de la maladie de Huntington selon la revendication 1, **caractérisée en ce que** le mécanisme de traitement provoque une augmentation de la viabilité cellulaire par une réduction considérable du niveau de huntingtine mutante dans les cellules.

3. Composition pharmaceutique contenant un composé actif 5,7-dihydroxy-3-(4-hydroxyphényl)-4H-1-benzopiran-4-one couplé à au moins un transporteur ou un solvant pharmaceutique pour utilisation dans le traitement de la maladie de Huntington provoquant une réduction du niveau d'agrégats de protéine huntingtine mutante dans les cellules par induction du processus d'autophagie.

4. La composition selon la revendication 3 pour une utilisation à la dose de 5-150 mg/kg/jour.

5. La composition selon la revendication 4 pour l'utilisation dans l'administration orale.
